# EUROPÄISCHE PATENTSCHRIFT

(11) **EP 0 507 896 B1**
(45) Veröffentlichungstag und Bekanntmachung des Hinweises auf die Patenterteilung: **02.08.1995**
(21) Anmeldenummer: 91914806.4
(22) Anmeldetag: 26.08.1991
(51) Int. Cl.: A61K 7/06

(54) **MITTEL ZUR FESTIGUNG DER HAARE**
HAIR-FIXING AGENT
PRODUIT POUR LA FIXATION DES CHEVEUX

(30) Priorität: 29.10.1990 DE 4034315
(43) Veröffentlichungstag der Anmeldung: 14.10.1992
(73) Patentinhaber: Wella Aktiengesellschaft, 64295 Darmstadt (DE)
(72) Erfinder: CLAUSEN, Thomas, D-6146 Alsbach (DE); HERFURT, Ulrich, D-6500 Mainz 31 (DE)
(86) Internationale Anmeldenummer: EP9101623
(87) Internationale Veröffentlichungsnummer: WO9207545

(56) Entgegenhaltungen:
- EP-A- 0 370 764
- BE-A- 793 589
- CA-A- 1 222 461
- GB-A- 902 808
- US-A- 4 397 836
- COSMETICS & TOILETRIES Bd. 94, Nr. 4, April 1979, Seiten 75 - 76; F.T. KOEHLER: 'CARBOXYLATED RESIN SYSTEMS IN HAIR CARE PRODUCTS.' siehe das ganze Dokument

## Beschreibung

Die Erfindung betrifft ein Mittel zur Festigung der Haare, welches eine Kombination aus einem teilweise oder vollständig neutralisierten amphoteren Polymer und einem nichtionischen Polymer enthält.

Mittel zur Festigung der Haare bestehen üblicherweise aus Lösungen von filmbildenden natürlichen oder synthetischen Polymeren. Als Polymer werden beispielsweise anionische Polymere wie Acrylsäure- oder Methacrylsäure-Homopolymere oder -Copolymere, Copolymerisate aus Acrylsäure und Acrylamiden und Copolymere auf der Basis von Alkylvinylethern und Maleinsäuremonoalkylestern, amphoteren Polymere wie Copolymerisate aus Octylacrylamid, Acrylat und Butylaminoethylmethacrylat, sowie nichtionische Polymere wie Vinylpyrrolidon-Homopolymere, Vinylpyrrolidon/Vinylacetat-Copolymere und Copolymerisate aus Vinylpyrrolidon, Vinylacetat und Vinylpropionat verwendet.

Für die Verwendung in Mitteln zur Festigung der Haare geeignete Polymere müssen eine Vielzahl von Eigenschaften besitzen. So müssen sie zum Beispiel in Alkoholen und Alkohol-Wasser-Gemischen gut löslich sowie mit den üblicherweise verwendeten Aerosol-Treibmitteln verträglich sein.

Weiterhin müssen sie auf dem Haar einen klaren, glänzenden Film bilden und dem Haar einen festen und zugleich flexiblen Halt geben ohne den natürlichen Griff des Haares zu beeinträchtigen. Ebenfalls müssen diese Polymeren gegen Luftfeuchtigkeit beständig sein, damit auch bei hoher Luftfeuchtigkeit ihre Festigungseigenschaften erhalten bleiben und das Haar sich nicht klebrig anfühlt. Gleichzeitig muß jedoch auch gewährleistet sein, daß diese Polymere leicht und rückstandsfrei aus dem Haar wieder ausgewaschen werden können.

Zur Erzielung der vorstehend aufgeführten Eigenschaften müssen diese Polymeren häufig mit bestimmten Zusatzstoffen, wie zum Beispiel Weichmachern oder hydrophobierenden Substanzen, kombiniert werden. Ebenfalls ist es üblich, bei freie Säuregruppen enthaltenden Polymeren die Auswaschbarkeit durch eine Neutralisierung der Säuregruppen zu verbessern.

Obwohl eine Vielzahl von natürlichen und synthetischen Polymeren mit unterschiedlichen Eigenschaften bekannt sind, ist es bisher nicht gelungen, ein Mittel zur Festigung der Haare zur Verfügung zu stellen, das in allen Eigenschaften - insbesondere aber bezüglich der Festigungswirkung, der Auswaschbarkeit sowie der Beständigkeit gegenüber Luftfeuchtigkeit - völlig zufriedenstellend ist.

Es bestand daher die Aufgabe, ein Mittel zur Festigung der Haare zur Verfügung zu stellen, daß die Nachteile der bisher bekannten Mittel nicht aufweist, das heißt sowohl eine gute Festigung der Haare bewirkt als auch betändig gegen Luftfeuchtigkeit ist und gleichzeitig aus dem Haar leicht ausgewaschen werden kann.

Überraschenderweise wurde nunmehr gefunden, daß durch die Kombination von mindestens einem teilweise oder vollständig neutralisierten amphoteren Polymer mit mindestens einem nichtionischen Polymer, ein Mittel zur Festigung der Haare erhalten wird, das alle an ein derartiges Mittel gestellten Anforderungen in hervorragender Weise erfüllt.

Gegenstand der vorliegenden Erfindung ist daher ein Mittel zur Festigung der Haare auf der Basis einer eine Polymerkombination enthaltenden alkoholischen oder wäßrig-alkoholischen Lösung, welches dadurch gekennzeichnet ist, daß die Polymerkombination aus (a) mindestens einem zu 50 bis 100 Prozent neutralisierten amphoteren Polymer und (b) mindestens einem nichtionischen Polymer besteht.

Als geeignete amphotere Polymere (a) können beispielsweise genannt werden:
(1) Copolymerisate eines Monomers, das sich von einer Vinylverbindung mit einer Carbonsäuregruppe ableitet, vorzugsweise Acryl-, Methacryl-, Malein- oder α-Chloressigsäure, mit einem basischen Monomer, das sich von einer substituierten Vinylverbindung mit mindestens einem basischen Stickstoffatom ableitet, vorzugsweise einem Dialkylaminoalkylmethacrylat oder -acrylat oder Dialkylaminoalkylmethacrylamid oder -acrylamid;
(2) Polymerisate mit Einheiten, die sich ableiten von
   - mindestens einem Monomer aus der Gruppe der Acrylamide und Methacrylamide, die am Stickstoff durch einen Alkylrest substituiert sind,
   - mindestens einem sauren Comonomer mit einer oder mehreren reaktiven Carbonsäuregruppen und
   - mindestens einem basischen Comonomer, beispielsweise Acryl- oder Methacrylsäureestern mit primären, sekundären, tertiären oder quaternären Aminosubstituenten oder dem Quaternisierungsprodukt von Dimethylaminoethylmethacrylaten mit Dimethyl- oder Diethylsulfat;
(3) Polymerisate mit zwitterionischen Einheiten der Formel 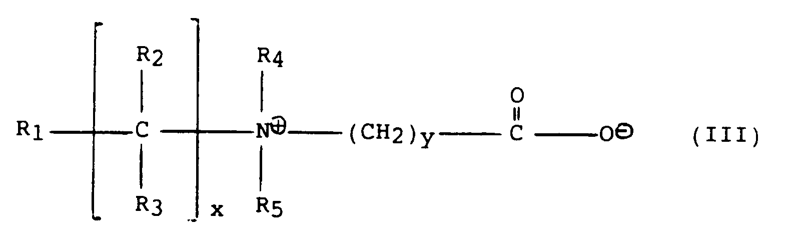 in der R₁, polymerisierbare ungesättigte Gruppe, zum Beispiel eine Acrylat-, Methacrylat-, Acrylamid- oder Methacrylamidgruppe ist,x und y ganze Zahlen von 1 bis 3 sind, R₂ und R₃ Wasserstoff, Methyl, Ethyl oder Propyl bedeuten, R₄ und R₅ Wasserstoff oder Alkylreste sind, wobei die Summe der Kohlenstoffatome in R₄ und R₅ jeweils nicht größer als 10 ist;
(4) Polymerisate, die sich von Chitosan ableiten und Monomereinheiten der folgenden Formeln enthalten: 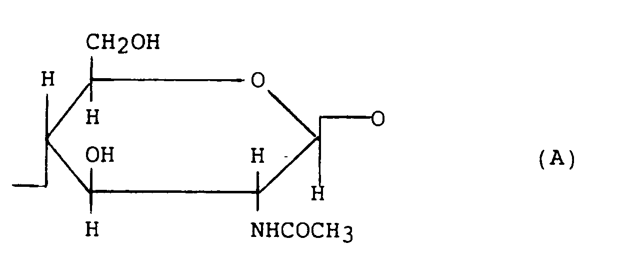 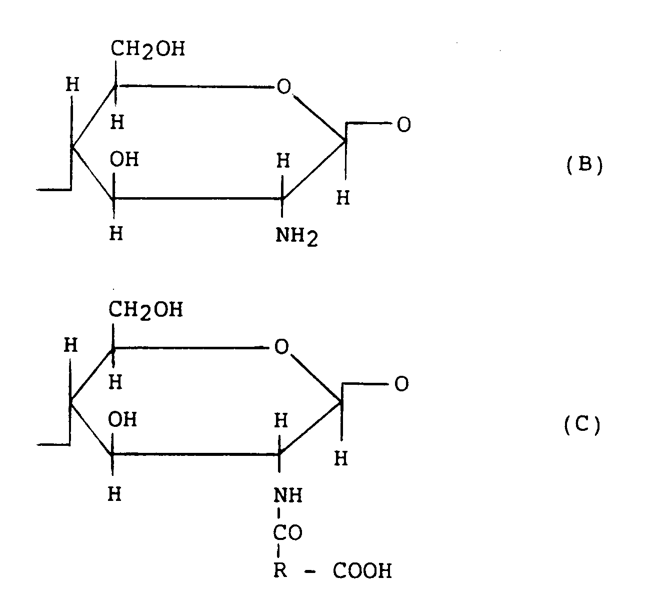 wobei die Einheit (A) in einem Anteil von 0 bis 30 %, die Einheit (B) in einem Anteil von 5 bis 50 % und die Einheit (C) in einem Anteil von 30 bis 90 % vorhanden ist und R in der Formel (C) einen Rest der Formel 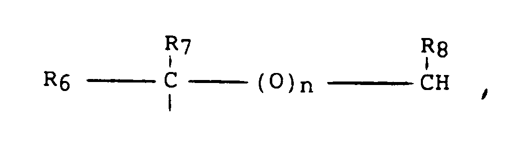 mit n = 0 oder 1,
   bedeutet, wobei im Falle von n = O die Reste R₆, R₇ und R₈ unabhängig voneinander Wasserstoff, Methyl, Hydroxyl, Acetoxy oder Amino, einen Monoalkylamin-oder Dialkylaminrest, der gegebenenfalls durch ein oder mehrere Stickstoffatome unterbrochen und/oder gegebenenfalls durch eine oder mehrere Amin-, Hydroxyl-, Carboxyl-, Alkylthio- oder Sulfonsäuregruppen substituiert ist, oder einen Alkylthiorest bedeuten, dessen Alkylgruppe einen Aminorest trägt, wobei mindestens einer der Reste R₆, R₇ und R₈ in diesem Fall Wasserstoff bedeutet, oder im Falle von n = 1 R₆, R₇ und R₈ jeweils Wasserstoffatome sind, sowie Salzen dieser Verbindungen mit Basen oder Säuren;
(5) Polymerisate der allgemeinen Formel 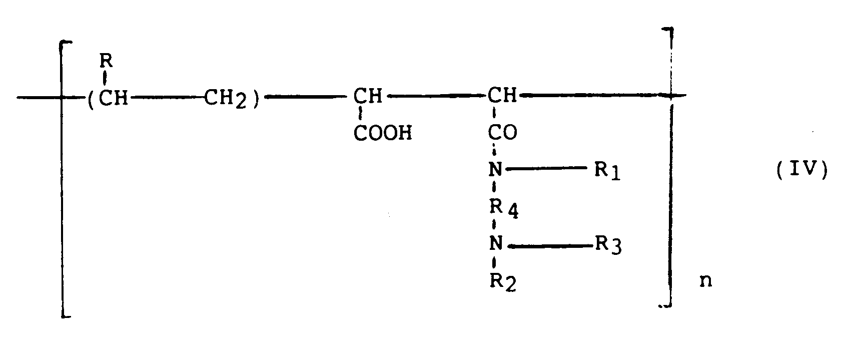 in der R Wasserstoff, CH₃O, CH₃CH₂O oder Phenyl bedeutet, R₁ Wasserstoff oder einen niederen Alkylrest, beispielsweise Methyl oder Ethyl, darstellt, R₂ Wasserstoff oder ein niederer Alkylrest, zum Beispiel ein Methyl- oder Ethylrest, ist, R₃ einen niederen Alkylrest, beispielsweise einen Methyl- oder Ethylrest, oder einen Rest der Formel R₄-N(R₂)₂ bedeutet und R₄ die Gruppe -CH₂-CH₂-, -CH₂-CH₂-CH₂-, 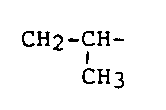 sowie die höheren Homologen dieser Reste darstellt und bis zu 6 Kohlenstoffatome enthält.

Unter diesen amphoteren Polymeren ist das Octylacrylamid/ Acrylat/Butylaminoethylmethacrylat-Copolymer, welches unter dem Namen AMPHOMER® von der National Starch & Chemical Corp., New Jersey/USA vertrieben wird, besonders bevorzugt.

Die vorstehend genannten amphoteren Polymere werden gemäß der Erfindung in einer zu 50 bis 100 Prozent durch organische Amine oder Alkanolamine neutralisierten Form eingesetzt.

Zur Neutralisierung geeignete Amine oder Alkanolamine sind zum Beispiel
2-Amino-2-methyl-1,3-propandiol,
2-Amino-2-ethyl-1,3-propandiol,
2-Amino-2-methyl-1-propanol,
2-Amino-2-methyl-2-pentanol,
1-Amino-2-methyl-2-propanol,
2-Amino-1-butanol,
3-Amino-2-pentanol,
2-Amino-1-phenyl-1-butanol,
2-Dimethylamino-2-methyl-1-propanol,
2-Dimethylamino-2-methyl-1,3-propandiol,
tris-(Hydroxymethyl)-aminomethan,
Triisopropylamin,
tris-(Hydroxymethyl)-dimethylaminomethan,
und
N¹-(2-Hydroxyethyl)-2-methyl-1,2-propandiamin,
wobei eine Neutralisierung mit 2-Amino-2-methyl-1-propanol, 2-Amino-1-butanol und Triisopropylamin besonders bevorzugt ist.

Als nichtionische Polymere (b) können beispielsweise Polyvinylpyrrolidon, Copolymere aus Vinylpyrrolidon und Vinylacetat oder Terpolymere aus Vinylpyrrolidon, Vinylacetat und Vinylpropionat verwendet werden.

Besonders bevorzugt ist gemäß der vorliegenden Erfindung die Verwendung von Copolymeren aus Vinylpyrrolidon und Vinylacetat, welche zum Beispiel von der Firma BASF, Ludwigshafen/BRD unter dem Namen LUVISKOL® VA vertrieben werden, sowie Terpolymeren aus Vinylpyrrolidon, Vinylacetat und Vinylpropionat, welche von der Firma BASF, Ludwigshafen/BRD unter dem Namen LUVISKOL® VAP vertrieben werden.

Das amphotere Polymer (a) und das nichtionische Polymer (b) sind in dem erfindungsgemäßen Mittel jeweils in einer Menge (bezogen auf die Festsubstanz) von 1 bis 11 Gewichtsprozent, vorzugsweise von 1 bis 6 Gewichtsprozent,enthalten, wobei die Gesamtmenge (bezogen auf die Festsubstanz) der beiden Polymere in dem erfindungsgemäßen Mittel 2 bis 12 Gewichtsprozent, vorzugsweise 4 bis 8 Gewichtsprozent, beträgt.

Vorzugsweise werden das amphotere Polymer (a) und das nichtionische Polymer (b) in dem erfindungsgemäßen Mittel in einem Gewichtsverhältnis (bezogen auf die Festsubstanz) von 1:4 bis 4:1 eingesetzt.

Das erfindungsgemäße Mittel zur Festigung der Haare weist vorzugweise einen pH-Wert von 7 bis 9,5 auf und kann in Form einer alkoholischen oder wäßrig-alkoholischen Lösung vorliegen.

Als Alkohol kommen hierbei insbesondere die für kosmetische Zwecke üblicherweise verwendeten niederen Alkohole mit 1 bis 4 Kohlenstoffatomen, wie zum Beispiel Ethanol und Isopropanol, in Betracht. Diese Alkohole können in dem erfindungsgemäßen Mittel in einer Menge von 10 bis 98 Gewichtsprozent, vorzugsweise in einer Menge von 20 bis 90 Gewichtsprozent, enthalten sein.

Selbstverständlich können in dem erfindungsgemäßen Mittel auch weitere haarkosmetische Zusätze, wie beispielsweise Parfümöle, Kräuterextrakte, bakterizide oder fungizide Stoffe, Lösungsvermittler für Parfümöle, Weichmacher wie Phthalsäureester oder Alkylzitrate, oder hydrophobierende Substanzen wie Siliconöle, enthalten sein, soweit derartige Zusätze nützlich und zweckmäßig erscheinen.

Das erfindungsgemäße Mittel zur Festigung der Haare liegt in der Regel in der Form eines Aerosol-Haarsprays oder Aerosol-Haarlackes beziehungsweise eines mit Hilfe einer geeigneten Sprühvorrichtung (zum Beispiel einer Sprühpumpe) versprühbaren Non-Aerosol-Haarsprays oder Non-Aerosol-Haarlackes vor.

Wenn das erfindungsgemäße Mittel in Form eines Aerosol-Haarsprays oder Aerosol-Haarlackes vorliegt, so enthält es zusätzlich 2 bis 80 Gewichtsprozent eines Treibmittels und wird in einen Druckbehälter abgefüllt. Als Treibmittel sind beispielsweise leicht flüchtige Fluorchlorkohlenwasserstoffe, wie zum Beispiel Difluorchlormethan oder Trichlormonofluormethan, Tetrafluordichlorethan oder niedere Alkane, wie zum Beispiel n-Butan, i-Butan und Propan, oder auch Dimethylether sowie ferner bei den in Betracht kommenden Drücken gasförmig vorliegende Treibmittel, wie beispielsweise N₂, N₂O und CO₂, sowie Gemische der vorstehend genannten Verbindungen geeignet. Unter den vorstehend genannten Treibmitteln sind Dimethylether sowie Mischungen von niederen Alkanen mit Dimethylether bevorzugt, wobei die Verwendung von Dimethylether als Treibmittel besonders bevorzugt ist.

Falls das erfindungsgemäße Mittel zur Festigung der Haare in Form eines Aerosol-Haarsprays oder Aerosol-Haarlackes vorliegt, bei dem als Treibmittel Dimethylether verwendet wird, so ist es auch möglich, daß das erfindungsgemäße Mittel in Form einer wäßrigen Lösung vorliegt, die eine Polymerkombination aus (a) mindestens einem zu 50 bis 100 Prozent neutralisierten amphoteren Polymer und (b) mindestens einem nichtionischen Polymer enthält.

Das Mittel zur Festigung der Haare gemäß der vorliegenden Erfindung bewirkt, bei gleichzeitig ausgezeichneter Festigung, eine Verbesserung von Glanz und Griff des Haares und ist leicht auswaschbar. Weiterhin fühlt sich das mit diesem Mittel behandelte Haar auch bei hoher Luftfeuchtigkeit nicht klebrig an.

Diese hervorragenden Eigenschaften beruhen auf einem synergistischen Effekt und waren aufgrund der Eigenschaften der eingesetzten Polymertypen nicht zu erwarten.

Die nachfolgenden Beispiele sollen den Gegenstand der Erfindung näher erläutern.

### Beispiele

### Beispiel 1: Aerosol Haarspray

3,0 g Octylacrylamid/Acrylat/Butylaminoethylmethacrylat-Copolymerisat (Amphomer® der National Starch & Chemical Corp./USA); zu 90 % mit 2-Amino-2-methyl-1-propanol neutralisiert
7,0 g Vinylpyrrolidon/Vinylacetat-Copolymer (Luviskol® VA 37 I der Firma BASF/BRD; 50 %-ige Lösung in Isopropanol)
0,2 g Parfümöl
0,1 g Diethylphthalat
28,7 g Ethanol
6,0 g Wasser
55,0 g Dimethylether
1̅0̅0̅,̅0̅ g̅

### Beispiel 2: Aerosol Haarspray

1,2 g Octylacrylamid/Acrylat/Butylaminoethylmethacrylat-Copolymerisat (Amphomer® der National Starch & Chemical Corp./USA); zu 100 % neutralisiert mit 2-Amino-1-butanol
9,6 g Vinylpyrrolidon/Vinylacetat-Copolymer (Luviskol® VA 28 E der Firma BASF/BRD; 50 %-ige Lösung in Ethanol)
0,2 g Parfümkomposition
0,1 g Triethylcitrat
29,4 g Ethanol
4,5 g Wasser
45,0 g Dimethylether
10,0 g Propan-/Butan-Gemisch
1̅0̅0̅,̅0̅ g̅

### Beispiel 3: Non-Aerosol Haarspray

6,4 g Octylacrylamid/Acrylat/Butylaminoethylmethacrylat-Copolymerisat (Amphomer® der National Starch & Chemical Corp./USA); zu 80 % neutralisiert mit Triisopropanolamin
3,2 g Vinylpyrrolidon/Vinylacetat/Vinylpropionat-Copolymer (Luviskol® VAP 343 I der Firma BASF/BRD; 50 %-ige Lösung in Isopropanol)
0,2 g Polyphenylmethylsiloxan
0,2 g Parfümöl
65,0 g Ethanol
25,0 g Isopropanol
1̅0̅0̅,̅0̅ g̅

### Beispiel 4: Aerosol Haarspray

3,5 g Octylacrylamid/Acrylat/Butylaminoethylmetha
crylat-Copolymerisat (Amphomer® der National Starch & Chemical Corp./USA); zu 85 % neutralisiert mit 2-Amino-2-methyl-1-propanol
9,0 g Vinylpyrrolidon/Vinylacetat-Copolymer (Luviskol® VA 37 E der Firma BASF/BRD; 50 %-ige Lösung in Ethanol)
0,2 g Parfümöl
26,3 g Ethanol
6,0 g Wasser
55,0 g Dimethylether
1̅0̅0̅,̅0̅ g̅

### Vergleichsversuche

Ein Aerosol Haarspray gemäß Beispiel 4 (Haarspray A) wurde bezüglich seiner Festigungseigenschaften, seiner Auswaschbarkeit sowie seiner Klebrigkeit mit zwei Haarsprays verglichen, die jeweils nur eines der beiden Polymere, entweder ein nichtionisches Polymer (Haarspray B) oder aber ein amphoteres Polymer (Haarspray C), enthielten.

### Haarspray B

16,0 g Vinylpyrrolidon/Vinylacetat-Copolymer (Luviskol® VA 37 E der Firma BASF/BRD; 50 %-ige Lösung in Ethanol)
0,2 g Parfümöl
28,8 g Ethanol
55,0 g Dimethylether
1̅0̅0̅,̅0̅ g̅

### Haarspray C

6,5 g Octylacrylamid/Acrylat/Butylaminoethylmethacrylat-Copolymerisat (Amphomer® der National Starch & Chemical Cop./USA); zu 85 % neutralisiert mit 2-Amino-2-methyl-1-propanol
0,2 g Parfümöl
32,3 g Ethanol
6,0 g Wasser
55,0 g Dimethylether
1̅0̅0̅,̅0̅ g̅

### Festigungseigenschaften

In einem Anwendungstest auf frisiertem Haar wurden die 3 Haarspray-Präparate hinsichtlich ihrer Festigungsstärke sowie der Haltbarkeit der Frisur miteinander verglichen. Hierzu wurden die Haare von jeweils 10 Versuchspersonen aus einer Entfernung von 30 cm mit dem Haarspray unter Verwendung eines üblichen Aerosolventil/Sprühkopf-Systems (mittlere Ausbringungsrate 0,6 g/s) 10 Sekunden lang besprüht.

Die Bewertung erfolgte durch eine friseur-fachliche Expertengruppe. Die in der nachfolgenden Tabelle angegebenen Bewertungen stellen den aus den Einzelbeurteilungen erhaltenen Mittelwert dar.

**Tabelle 1**

| | Festigungsstärke | Haltbarkeit der Frisur |
|---|---|---|
| Haarspray A | sehr gut bis gut | sehr gut bis gut |
| Haarspray B | befriedigend | gut bis befriedigend |
| Haarspray C | gut | gut |

### Klebrigkeit

Die Klebrigkeitsneigung der 3 Haarsprays wurde auf Glasplatten anhand einer standardisierten Fingerdruckmethode ermittelt.

**Tabelle 2**

| | |
|---|---|
| Haarspray A | nicht klebrig |
| Haarspray B | leicht klebrig |
| Haarspray C | nicht klebrig |

### Auswaschbarkeit

Die Auswaschbarkeit der 3 Haarsprays wurde anhand von Haarsträhnen, welche mehrmals mit dem Haarspray eingesprüht und zwischendurch getrocknet worden waren, verglichen. Die einzelnen Haarsträhnen wurden jeweils 2 Minuten lang mit 50 ml 35 °C warmem Wasser unter Verwendung von 0,2 g eines üblichen Haarshampoos gewaschen. Im Anschluß an diese Haarwäsche wurden die Haarsträhnen getrocknet und die Auswaschbarkeit der 3 Haarsprays anhand von Griff und Weichheit der Haare sowie des Vorhandenseins von sichtbaren Rückständen beurteilt. Das Ergebnis wird in Tabelle 3 zusammengefaßt.

**Tabelle 3**

| | |
|---|---|
| Haarspray A | sehr gute bis gute Auswaschbarkeit |
| Haarspray B | sehr gute Auswaschbarkeit |
| Haarspray C | gute Auswaschbarkeit |

Durch die vorstehenden Vergleichsversuche wird eindeutig belegt, daß das erfindungsgemäße Haarspray die Vorteile von nichtionischen beziehungsweise amphoteren Polymere enthaltenden Haarsprays vereinigt ohne jedoch deren Nachteile aufzuweisen.

Alle Prozentangaben in der vorliegenden Anmeldung stellen Gewichtsprozent dar.

## Patentansprüche

1. Mittel zur Festigung der Haare auf der Basis einer eine Polymerkombination enthaltenden alkoholischen oder wäßrig-alkoholischen Lösung, dadurch gekennzeichnet, daß die Polymerkombination aus (a) mindestens einem zu 50 bis 100 Prozent neutralisierten amphoteren Polymer und (b) mindestens einem nichtionischen Polymer besteht.

2. Mittel nach Anspruch 1, dadurch gekennzeichnet, daß es die Polymerkombination in einer Menge von 2 bis 12 Gewichtsprozent enthält.

3. Mittel nach Anspruch 1 oder 2, dadurch gekennzeichnet, daß es das amphotere Polymer in einer Menge von 1 bis 11 Gewichtsprozent enthält.

4. Mittel nach einem der Ansprüche 1 bis 3, dadurch gekennzeichnet, daß es das nichtionische Polymer in einer Menge von 1 bis 11 Gewichtsprozent enthält.

5. Mittel nach einem der Ansprüche 1 bis 4, dadurch gekennzeichnet, daß das Gewichtsverhältnis von amphoterem Polymer zu nichtionischem Polymer 1:4 bis 4:1 beträgt.

6. Mittel nach einem der Ansprüche 1 bis 5, dadurch gekennzeichnet, daß das amphotere Polymer ein Octylacrylamid/Acrylat/Butylaminoethylmethacrylat-Copolymer ist.

7. Mittel nach einem der Ansprüche 1 bis 6, dadurch gekennzeichnet, daß das amphotere Polymer mit 2-Amino-2-methyl-1,3-propandiol, 2-Amino-2-ethyl-1,3-propandiol, 2-Amino-2-methyl-1-propanol, 2-Amino-2-methyl-2-pentanol, 1-Amino-2-methyl-2-propanol, 2-Amino-1-butanol, 3-Amino-2-pentanol, 2-Amino-1-phenyl-1-butanol, 2-Dimethylamino-2-methyl-1-propanol, 2-Dimethylamino-2-methyl-1,3-propandiol, tris-(Hydroxymethyl)-dimethylaminomethan,Triisopropylamin, tris-(Hydroxymethyl)-aminomethan oder N¹-(2-Hydroxyethyl)-2-methyl-1,2-propandiamin neutralisiert ist.

8. Mittel nach einem der Ansprüche 1 bis 7, dadurch gekennzeichnet, daß das nichtionische Polymer ausgewählt ist aus Polyvinylpyrrolidon; Copolymeren aus Vinylpyrrolidon und Vinylacetat; und Terpolymeren aus Vinylpyrrolidon, Vinylacetat und Vinylpropionat.

9. Mittel nach einem der Ansprüche 1 bis 8, dadurch gekennzeichnet, daß es 2 bis 80 Gewichtsprozent eines Treibmittels enthält und in Form eines Aerosol-Haarsprays oder - Haarlackes vorliegt.

10. Mittel nach einem der Ansprüche 1 bis 8, dadurch gekennzeichnet, daß es in Form eines Non-Aerosol-Haarsprays oder - Haarlackes vorliegt.

11. Mittel zur Festigung der Haare auf der Basis einer eine Polymerkombination enthaltenden wäßrigen Lösung, welches als Treibmittel Dimethylether enthält und in Form eines Aerosol-Haarsprays oder -Haarlackes vorliegt, dadurch gekennzeichnet, daß die Polymerkombination aus (a) mindestens einem zu 50 bis 100 Prozent neutralisierten amphoteren Polymer und (b) mindestens einem nichtionischen Polymer besteht.

## Claims

1. Hair setting agent based on an alcoholic or aqueous-alcoholic solution containing a polymer combination, characterised in that the polymer combination consists (a) of at least one 50 to 100 % neutralised amphoteric polymer and (b) of at least one non-ionic polymer.

2. Agent according to Claim 1, characterised in that it contains the polymer combination in an amount of between 2 and 12 weight %.

3. Agent according to Claim 1 or 2, characterised in that it contains the amphoteric polymer in an amount of between 1 and 11 weight %.

4. Agent according to any one of Claims 1 to 3, characterised in that it contains the non-ionic polymer in an amount of between 1 and 11 weight %.

5. Agent according to any one of Claims 1 to 4, characterised in that the weight ratio of amphoteric Polymer to non-ionic polymer is from 1:4 to 4:1.

6. Agent according to any one of Claims 1 to 5, characterised in that the amphoteric polymer is an octylacrylamide/acrylate/butylaminoethylmethacrylate copolymer.

7. Agent according to any one of Claims 1 to 6, characterised in that the amphoteric polymer is neutralised with 2-amino-2-methyl-1,3-propanediol, 2-amino-2-ethyl-1,3-propanediol, 2-amino-2-methyl-1-propanol, 2-amino-2-methyl-2-pentanol, 1-amino-2-methyl-2-propanol, 2-amino-1-butanol, 3-amino-2-pentanol, 2-amino-1-phenyl-1-butanol, 2-dimethylamino-2-methyl-1-propanol, 2-dimethylamino-2-methyl-1,3-propanediol, tris-(hydroxymethyl)-dimethylamino-methane, triisopropylamine, tris-(hydroxymethyl)-aminomethane or N¹-(2-hydroxyethyl)-2-methyl-1,2-propanediamine.

8. Agent according to any one of Claims 1 to 7, characterised in that the non-ionic polymer is selected from polyvinylpyrrolidone; copolymers of vinylpyrrolidone and vinylacetate; and terpolymers of vinylpyrrolidone, vinylacetate and vinylpropionate.

9. Agent according to any one of Claims 1 to 8, characterised in that it contains from 2 to 80 weight % of a propellant and is in the form of an aerosol hairspray or aerosol hair lacquer.

10. Agent according to any one of Claims 1 to 8, characterised in that it is present in the form of a non-aerosol hairspray or non-aerosol hair lacquer.

11. Hair setting agent based on an aqueous, polymer combination-containing solution which contains dimethylether as propellant and is present in the form of an aerosol hairspray or aerosol hair lacquer, characterised in that the polymer combination consists (a) of at least one 50 to 100 % neutralised amphoteric polymer and (b) of at least one non-ionic polymer.

## Revendications

1. Produit de fixation des cheveux, à base d'une solution alcoolique ou d'une solution aqueuse-alcoolique contenant une combinaison de polymères, caractérisé en ce que la combinaison de polymères est composée de (a) au moins un polymère amphotère, neutralisé à 50 à 100 %, et (b) au moins un polymère non ionique.

2. Produit sel on la revendication 1, caractérisé en ce qu'il contient la combinaison de polymères en une quantité de 2 à 12 pourcent en poids.

3. Produit selon la revendication 1 ou 2, caractérisé en ce qu'il contient le polymère amphotère en une quantité de 1 à 11 pourcent en poids.

4. Produit selon l'une des revendications 1 à 3, caractérisé en ce qu'il contient le polymère non ionique en une quantité de 1 à 11 pourcent en poids.

5. Produit selon l'une des revendications 1 à 4, caractérisé en ce que le rapport de poids entre le polymère amphotère et le polymère non ionique est de 1:4 à 4:1.

6. Produit selon l'une des revendications 1 à 5, caractérisé en ce que le polymère amphotère est un copolymère octylacrylamide/acrylate/butylaminoéthylméthacrylate

7. Produit sel on lune des revendications 1 à 6, caractérisé en ce que le polymère amphotère est neutralisé avec le 2-amino-2-méthyl-1,3-propanediol, 2-amino-2-ethyl-1,3-propanediol, 2-amino-2-methyl-1-propanol, 2-amino-2-methyl-2-pentanol, 1-amino-2-methyl-2-propanol, 2-amino-1-butanol, 3-amino-2-pentanol, 2-amino-1-phenyl-1-butanol, 2-dimethylamino-2-methyl-1-propanol, 2-dimethylamino-2-methyl-1,3-propandiol, tris-(hydroxymethyl)-dimethylaminométhane, triisopropylamine, tris-(hydroxymethyl)-aminométhane ou N¹-(2-hydroxyethyl)-2-methyl-1,2-propandiamine.

8. Produit sel on l'une des revendications 1 à 7, caractérisé en ce que le polymère non ionique est sélectionné parmi le polyvinylpyrrolidone ; les copolymères de vinylpyrrolidon et l'acétate de vinyl; et les terpolymères parmi le vinylpyrrolidone, l'acétate de vinyl et le propionate de vinyl.

9. Produit sel on l'une des revendications 1 à 8, caractérisé en ce qu'il contient de 2 à 80 pourcent en poids d'un produit moussant et se présente sous la forme d'un produit aérosol à pulvériser ou sous forme de laque, pour les cheveux.

10. Produit selon l'une des revendications 1 à 8, caractérisé en ce qu'il se présente sous la forme d'un produit non aérosol à pulvériser ou sous forme de laque, pour les cheveux.

11. Produit de fixation des cheveux, à base d'une solution aqueuse contenant une combinaison de polymères, contenant comme produit moussant du dymétylether et se présentant sous la forme d'un produit aérosol à pulvériser ou sous forme de laque, pour les cheveux, caractérisé en ce que la combinaison de polymères est composée de (a) au moins un polymère amphotère, neutralisé à 50 à 100 %, et (b) au moins un polymère non ionique.
